# EUROPEAN PATENT SPECIFICATION

(11) **EP 4 419 051 B1**
(45) Date of publication and mention of the grant of the patent: **12.08.2026**
(21) Application number: 22809578.2
(22) Date of filing: 20.10.2022
(51) Int. Cl.: A61F 5/44, A61F 5/441, A61F 5/445

(54) **DEVICES, SYSTEMS AND METHODS FOR REGULATING FLOW FROM A STOMA ON A PATIENT**
VORRICHTUNGEN, SYSTEME UND VERFAHREN ZUR REGULIERUNG DES DURCHFLUSSES AUS EINEM STOMA EINES PATIENTEN
DISPOSITIFS, SYSTEMES ET METHODES DE REGULATION DE L'ECOULEMENT D'UN STOMA D'UN PATIENT

(30) Priority: 22.10.2021 US 202163270808 P; 22.06.2022 US 202217846863
(43) Date of publication of application: 28.08.2024
(73) Proprietor: OstoValve LLC, Richmond, VT 05477 (US)
(72) Inventor: BELL JR., Robert C., Richmond, VT 05477 (US); SHULOCK, Damien, Richmond, VT 05477 (US); MARGOLIS, Gary, Richmond, VT 05477 (US); ASHLEY, John, Richmond, VT 05477 (US)
(74) Representative: KIPA AB
(86) International application number: PCT/US2022/047332
(87) International publication number: WO 2023/069659

(56) References cited:
- BE-A- 850 143
- ES-A1- 2 804 033
- US-A1- 2002 077 611
- US-A1- 2010 241 092
- US-A1- 2019 380 860

## Description

### FIELD OF THE DISCLOSURE

The present disclosure generally relates to the field of osteotomy appliances. In particular, the present disclosure is directed to devices, systems and methods for regulating flow from a stoma on a patient.

### BACKGROUND

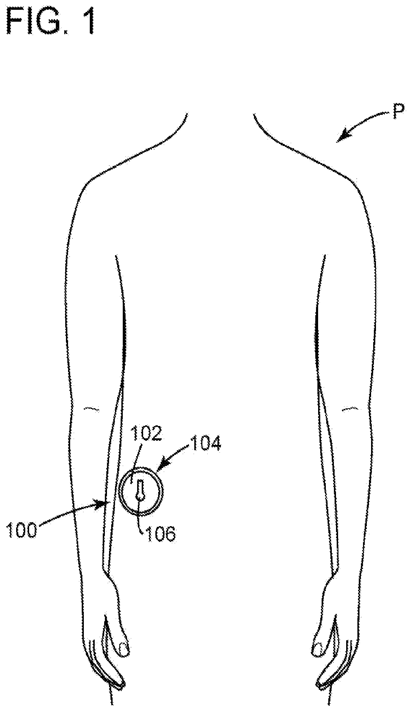

Surgical procedures often require post-operative access to a patient's body cavity to drain fluids or waste that may cause infection. A "stoma" is an example of one such type of surgically created access. In one common application a stoma is an opening to allow waste from the patient's intestines to exit the body following colostomy or ileostomy surgery. The waste collects in a device, like a bag or pouch, that attaches around the stoma or connects to the stoma via a drain tube. This conventional arrangement, while effective to allow drainage, typically affords the patient little control of waste as it drains involuntarily into a collection device attached to the drain tube. Patients may elect to occlude the drain passage with, for example, a plug (in the stoma) or clamp (on the drain tube), however, discomfort may result because the stoma is not meant to tolerate occlusion for long periods of time. Damage to the drain tube may also allow waste to leak before the collection device. In patent publication BE 850 143 A (COLOPLAST INTERNAT A S), on which the preamble of claim 1 is based, a stoma valve appliance, see in particular figure 1 thereof.
Further background documents disclosing devices of the field of the disclosure include ES 2 804 033 A1, US 2002/077611 A1, US 2010/241092 A1, and US 2019/380860 A1.

### SUMMARY OF THE DISCLOSURE

In one implementation, the present disclosure is directed to a stoma valve appliance, which includes an annular support member configured to surround a patient stoma and having an inner, skin facing side and an outer side; a base member removably attachable over a center of the annular support member, the base member defining a first solids and liquids flow path and a second gas vent flow path through the base member, each the flow path having at least one opening on an inner side of the base member; a valve disposed in the base member configured to control flow through the first flow path, the valve having an open position permitting flow and a closed position preventing flow and being manipulable between the open and closed position by the patient; a stem with an inlet and an outlet projecting inwardly from the body member configured to be received in the patient stoma, the stem having sufficient length to extend through the center of the annular support member to position the inlet in the stoma when the base member is mounted on the annular support member with the outlet at an opposite end communicating with the first flow path opening in the base member; and a gas permeable filter seal around the stem covering the at least one second gas vent flow path opening, whereby gasses from the stoma may be vented through the second flow path, while liquids and solids are prevented from entering the second flow path.

In another implementation, the present disclosure is directed to a patient controllable method for evacuating waste from a stoma. The method includes positioning a vented valve appliance in the stoma, the valve appliance having a closed position preventing waste from exiting the stoma and an open position allowing waste to exit the stoma; the patient selectively moving the valve appliance between the open and closed positions to evacuate waste from the stoma or retain waste within the stoma for later evacuation; and continuously venting gasses from the stoma through the valve appliance in both the open and closed positions.

In yet another implementation, the present disclosure is directed to a stoma valve appliance, which includes an annular support member configured to surround a patient stoma and having an inner, skin facing side and an outer side; a skin adhesion layer disposed on the annular support member inner, skin facing side; a base member removably attachable over a center of the annular support member, the base member defining a flow path through the base member, the flow path having an opening on an inner side of the base member; a valve disposed in the base member configured to control flow through the flow path, the valve having an open position permitting flow and a closed position preventing flow and being manipulable between the open and closed position by the patient; and an exit nozzle terminating the first path through the base member opposite the inner side opening receiving flow through the valve in the open position.

### BRIEF DESCRIPTION OF THE DRAWINGS

For the purpose of illustrating the disclosure, the drawings show aspects of one or more embodiments of the disclosure. However, it should be understood that the present disclosure is not limited to the precise arrangements and instrumentalities shown in the drawings, wherein:
- FIG. 1: depicts a sketch of an exemplary embodiment of a valve appliance in position on a patient;
- FIG. 2A: is a perspective view of an embodiment of a valve appliance.
- FIG. 2B: is a front view of the embodiment of the valve appliance shown in FIG. 2A.
- FIG. 2C: is a cross-sectional view of the embodiment of the valve appliance shown in FIG. 2B through line A-A.
- FIG. 3A: is an exploded perspective view of the embodiment of the valve appliance shown in FIG. 2A.
- FIG. 3B: is an exploded cross-sectional view of the embodiment of the valve appliance shown in FIG. 3A through line A-A (as shown in FIG. 2B).
- FIG. 4A: is a front view of some of the components in a closed configuration of the embodiment of the valve appliance shown in FIG. 2A.
- FIG. 4B: is a front view of some of the components in an open configuration of the embodiment of the valve appliance shown in FIG. 2A.
- FIG. 5: is a cross-sectional view of some of the components of the embodiment of the valve appliance of FIG. 2A in the open configuration of FIG. 4B through line A'-A'.
- FIG. 6A: is a front perspective view of an effluent capture device to be used with the embodiment of the valve appliance shown in FIG. 2A.
- FIG. 6B: is a rear perspective view of an effluent capture device shown in FIG. 6A.
- FIG. 7 is: a perspective view of an effluent capture device shown in FIG. 6A combined with the embodiment of the valve appliance shown in FIG. 2A.
- FIG. 8 is: a perspective view of some of the components of an effluent capture device shown in FIG. 6A combined with some of the components of the embodiment of the valve appliance shown in FIG. 2A.
- FIG. 9A: is a perspective view of another embodiment of a valve appliance.
- FIG. 9B: is a front view of the embodiment of the valve appliance shown in FIG. 9A.
- FIG. 9C: is a cross-sectional view of the embodiment of the valve appliance shown in FIG. 9B through line B-B.
- FIG. 9D: is a cross-sectional view of the valve appliance as shown in FIGS. 9A and 9B with an alternative filter-seal arrangement.
- FIG. 9E: is a cross-sectional view of an alternative valve appliance similar to the embodiment shown in FIG. 9A, but with no stem or gas vent.
- FIG. 10A: is a front view of some of the components in a closed configuration of the embodiment of the valve appliance shown in FIG. 9A.
- FIG. 10B: is a front view of some of the components in an open configuration of the embodiment of the valve appliance shown in FIG. 9A.
- FIG. 11A: is a rear view of the alternate effluent capture device clip.
- FIG. 11B: is a side view of the alternate effluent capture device clip shown in FIG. 11A.
- FIG. 12: is a perspective view of the valve appliance shown in FIG. 9A combined with the alternate effluent capture device clip shown in FIGS. 11A-B.
- FIG. 13A: is a perspective view of yet another embodiment of a valve appliance.
- FIG. 13B: is a front view of the embodiment of the valve appliance shown in FIG. 13A.
- FIG. 13C: is a cross-sectional view of the embodiment of the valve appliance shown in FIG. 13B through line C-C.
- FIG. 13D: is a cross-sectional view of the embodiment of the valve appliance shown in FIG. 13B through line D-D.
- FIG. 13E: is a cross-sectional view of the embodiment of the valve appliance shown in FIG. 13B through line E-E.
- FIG. 14A: is a perspective view of the embodiment of the valve appliance shown in FIG. 13A in an open configuration.
- FIG. 14B: is a front view of the embodiment of the valve appliance shown in FIG. 14A.
- FIG. 14C: is a cross-sectional view of the embodiment of the valve appliance shown in FIG. 14B through line F-F.

Where applicable, like-reference characters designate identical or corresponding components and units throughout the several views (which are not to scale unless otherwise indicated). The embodiments disclosed herein may include elements that appear in one or more of the several views or in combinations of the several views. Methods are exemplary only and may be modified by, for example, reordering, adding, removing, and/or altering the individual stages

### DETAILED DESCRIPTION

The discussion that follows describes medical appliances that can engage with surgically-formed stoma on a patient. These appliances allow patients to periodically discharge waste from their bodies into a bag or a container for proper disposal. The designs proposed allow the patient to control flow in a convenient manner while remaining properly engaged with the stoma to minimize leaks or other inadvertent discharge of waste.

FIG. 1 depicts a sketch diagram of an exemplary embodiment of a valve appliance 100. This embodiment is shown on a patient P, typically at a location on patient P where access to a body cavity is necessary after a surgical procedure. This location may have a stoma, or port, which allows waste to exit patient P into a collection device like a bag or pouch (not shown). The appliance 100 may include a support unit 102 that receives a spigot 104. A seal unit 106 may reside on part of the support unit 102 to engage with the interior of the stoma.

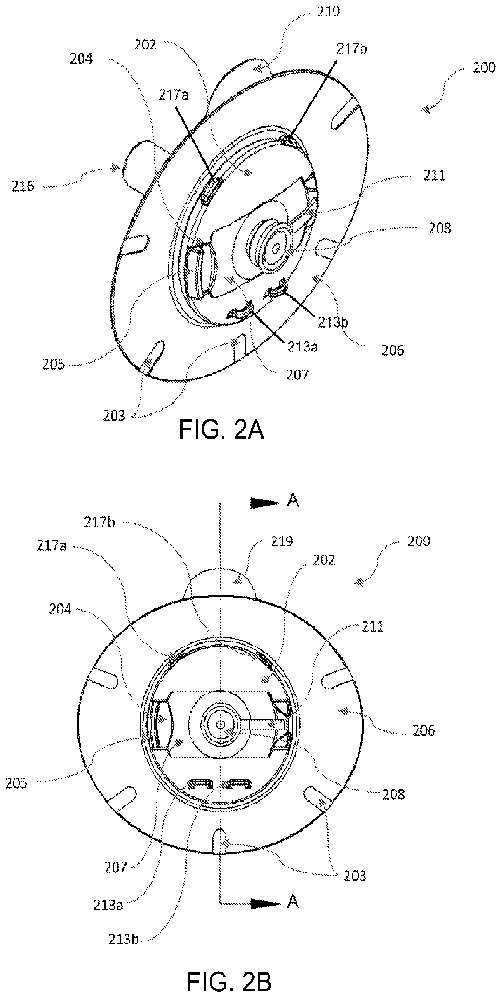

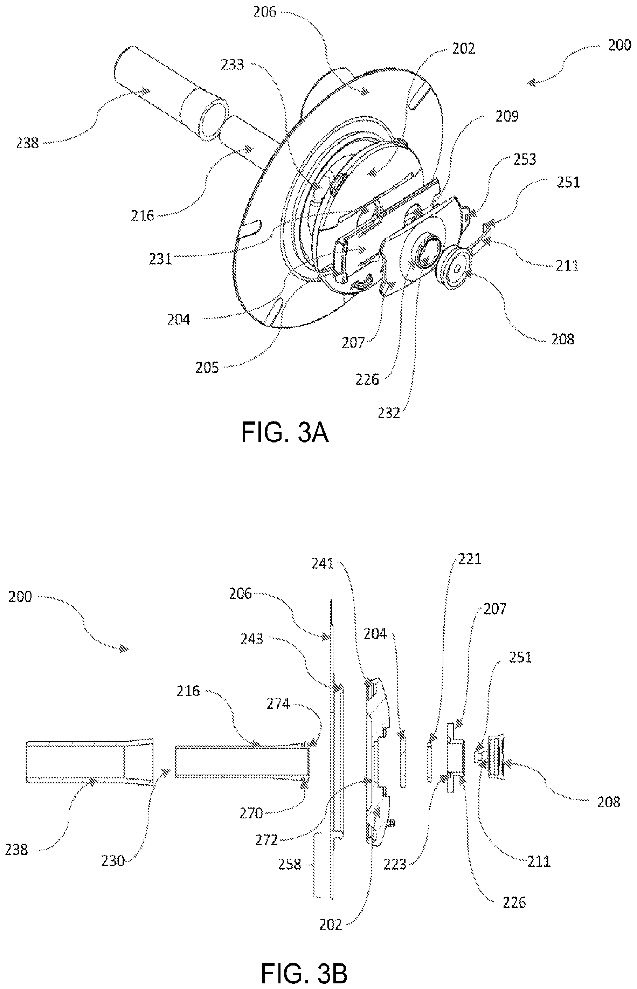

FIGS. 2A and 2B depict an alternative embodiment of a valve appliance 200 configured to interface with a patient's stoma to allow patient-controlled elimination of waste through the stoma. The valve appliance 200 is comprised of a base 202 that is attached to an adhesive wafer 206, which may be formed as a relatively thin, flexible annular disk. The adhesive wafer 206 is configured with adhesive on the back side 212 for securing the valve appliance 200 to a patient's skin. For example, an adhesive surface 258 (FIG. 3B) on the adhesive wafer 206 bonds the adhesive wafer 206 to the skin. Adhesive surface 258 can be comprised of any type of skin compatible adhesive such as a colloidal hydrogel adhesive. The adhesive wafer 206 can also include a removal tab 219 used to assist the patient when they want to remove the adhesive backed wafer from their skin and flex joints 203 that allow the adhesive wafer 206 to closely conform to the irregular contours of the patient's skin. The base 202 has one or more bag clip engagement slots 213a and 213b and one or more bag clip engagement tabs 217a and 217b. The bag clip engagement slots 213a and 213b and bag clip engagement tabs 217a and 217b are configured to hold a bag clip as will be described later herein. The base 202 engages a gate valve 204 that sits on the front of the base 202. The gate valve 204 has a valve handle 205 on one end to enable the patient to open and close the gate valve 204 as will be described later herein. The gate valve 204 is constrained to the base 202 by a valve cover 207. The valve cover 207 and base 202 constrain the gate valve 204 but allow the gate valve 204 to slide open and closed as will be described further herein. The valve cover 207 includes an exit nozzle 226 that guides effluent away from the valve appliance 200. The exit nozzle 226 can be covered by a nozzle cap 208 when the patient is not operating the valve appliance 200 to remove effluent. The nozzle cap 208 is connected to the base portion 202 by means of a tether 211. The tether 211 keeps the nozzle cap 208 connected to the valve appliance 200 when the patient removes the nozzle cap 208 from the exit nozzle 226.

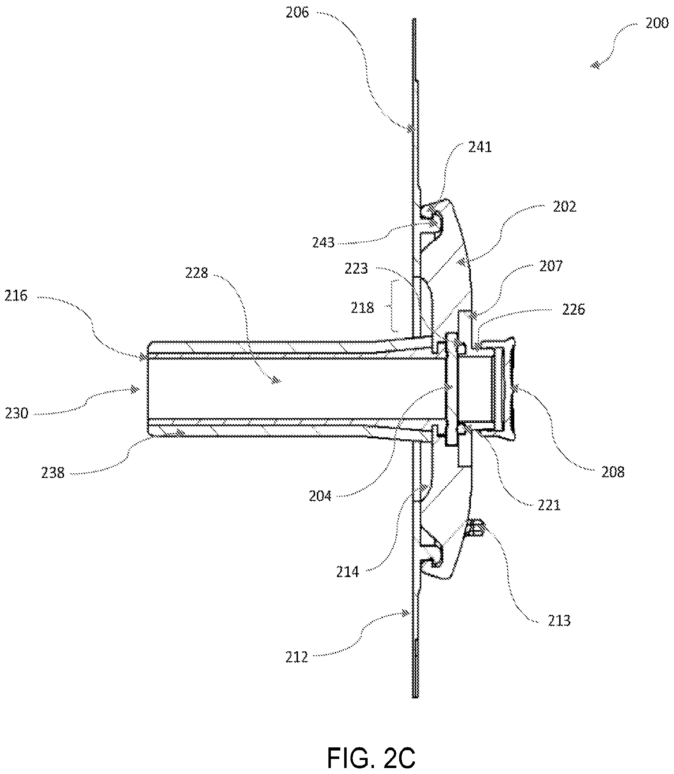

FIG. 2C depicts how the base portion 202 is engaged with the adhesive wafer 206 by a base clip 241 that engages with a wafer clip 243. Using a base clip 241 on the base portion 202 and wafer clip 243 on the adhesive wafer 206 allows the two parts to be connected and to separate as needed. For example, the patient might want to attach the base 202 to a smaller diameter adhesive wafer 206 that conforms better to their skin than the standard diameter adhesive wafer 206. Likewise, the patient might want to attach the base portion 202 to an adhesive wafer 206 that has a different type of adhesive that is more compatible with the patient's skin. Compatibility of the adhesive to the patient's skin is a very important characteristic of the valve appliance 200 as the patient will keep the valve appliance 200 attached to their skin for 2 or more days before removing the valve appliance 200 to replace it with a new one. The adhesive wafer 206 is also comprised of an annual opening 218, and the base 202 is comprised of a domed cavity 214. The annular opening 218 and domed cavity 214 are configured to cover the patient's stoma that often protrudes from the surface of the patient's skin without compressing or otherwise impacting the stoma. It is important to accommodate the stoma without irritating it by providing a cavity comprised of the annular opening 218 and domed cavity 214. The patient's stoma can change size and shape over time so allowing the patient to interchange the adhesive wafer 206 for different sizes and/or adhesive types. Alternatively, in some embodiments, adhesive wafer 206 may be configured to allow the patient to trim the annular opening to accommodate the patient's particular stoma size and shape. Similarly, the base 202 may be exchanged with other bases to provide a domed cavity 214 to best match the stoma shape and size. The two-piece configuration disclosed herein thus provides further advantages in patient comfort based on the patient having the ability to control and change shapes and adhesion types over time without needing to replace the entire device.

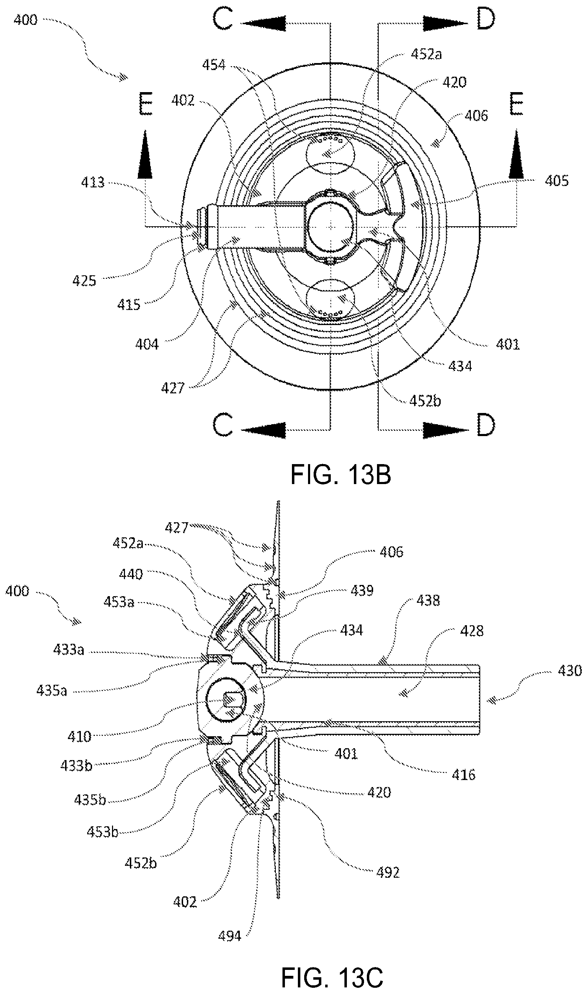

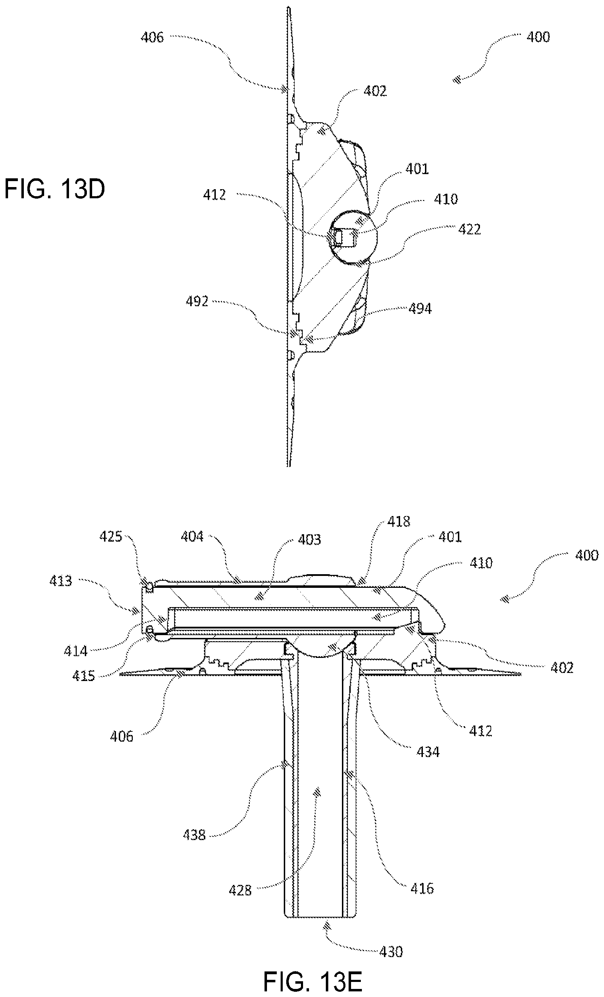

The base clip 241 and wafer clip 243 are not the only possible configuration for removably connecting the base 202 and adhesive wafer 206. In one alternative, the base clip 241 and wafer clip 243 could be replaced with interlocking threads allowing the base 202 and adhesive wafer 206 to be screwed together and screwed apart. If threads are used rather than the base clip 241 and wafer clip 243 then a detent, latch, compression washer, or other restraining device may be required in addition to the threads to prevent the threads from separating unless intended. An arrangement of interlocking ribs and grooves, such as shown in FIG. 13D and described below, also may be used. Other removeable connectors such as hook and loop fastening devices and other snap or interference fit devices also may be employed.

The valve cover 207 is configured with an annular recess 223 that is configured to hold an annular seal 221. The annular seal 221 is compressed against the valve cover 207 and the valve gate 204 to ensure that no effluent leaks between the valve cover 207 and the valve gate 204. The annular seal 221 could be an O-ring type seal. The annular seal 221 could also be any other type of seal as are commonly used to prevent fluid leakage between two parts. The annular seal 221 could also be adhesively or otherwise bonded to the valve cover 207 rather than compressed against the valve cover 207. The annular seal 221 could also be molded onto the valve cover 207.

On the back side of the valve appliance 200 there is a stem 216 that is covered by a gas permeable filter 238. The back end of the stem 216 has an entry port 230 that allows effluent to enter the drain passage 228. As shown in FIG. 2C, the valve 204 is in the closed position and as such is blocking the drain passage 228 and preventing effluent that enter the drain passage 228 from exiting the patient's stoma. The gas permeable filter 238 is configured to be compressed against the inside diameter of the stoma and prevent effluent from leaking between the outer surface of the gas permeable filter 238 and the stoma.

The front of the stem 216 is comprised of a slot 270 and a protrusion 274 as depicted in FIG. 3B. The slot 270 is configured to interface with a ledge 272 on the base 202 and thereby connect the stem 216 to the base 202. This connection between the slot 270 and the ledge 272 can be a simple overlapping connection. The connection can also include adhesive or similar bonding between the slot 270 and the ledge 272. The stem 216 could also be molded onto the base 202. The protrusion 274 is configured to seal against the backside of the gate valve 204 to prevent effluent in the drain passage 228 from leaking around the gate valve 204.

Referring to FIG. 3A, the valve cover 207 is shown with a tether slot 253 configured to receive and constrain a tether tab 251 on the end of the tether 211 opposite the nozzle cap 208. Although the tether slot 253 is shown as part of the valve cover 207 it could also be configured to be part of the base 202. A tether slot 253 and tether tab 251 are just one possible method for constraining the nozzle cap 208 and tether 211 to the rest of the valve appliance 200. The tether 211 could be adhesively or otherwise bonded to the valve cover 207 or to the base 202. The tether 211 could also be molded to the valve cover 207 or to the base 202.

The base 202 is shown with a base opening 231 that is aligned with the drain passage 228. The base opening 231 is also aligned with a valve cover opening 232 at the front end of the exit nozzle 226 on the valve cover 207. The gate valve 204 also has a gate opening 209. The entry port 230, drain passage 228, base opening 231 and valve cover opening 232 are all aligned to allow effluent to exit through the valve appliance 100. However, in the configuration depicted in FIGS. 2A-C and 3A-B, the gate opening 209 is not aligned with the entry port 230, drain passage 228, base opening 231 and valve cover opening 232 and the valve appliance 200 is closed by the gate valve 204.

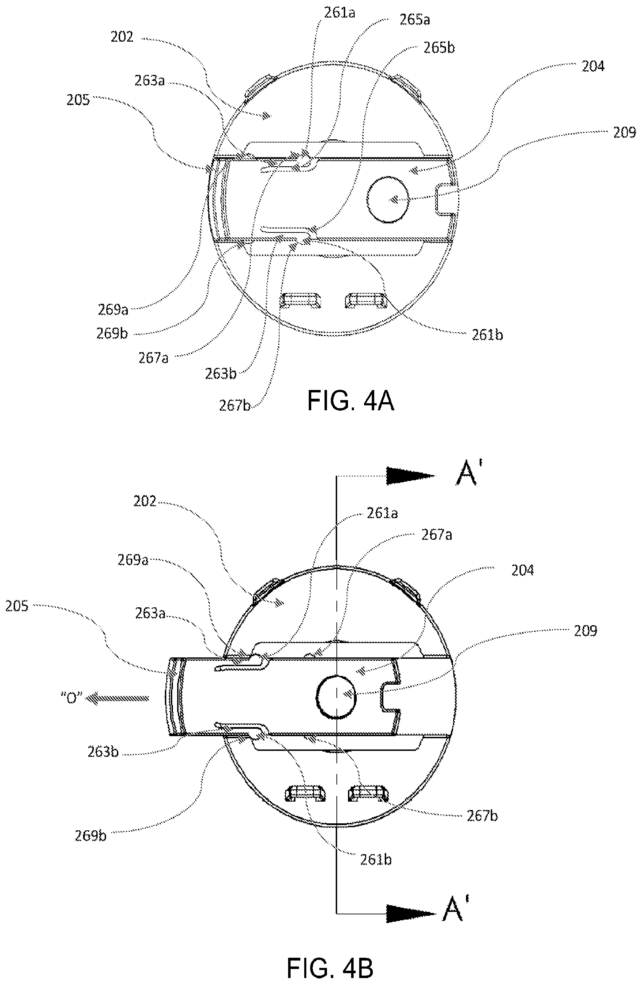

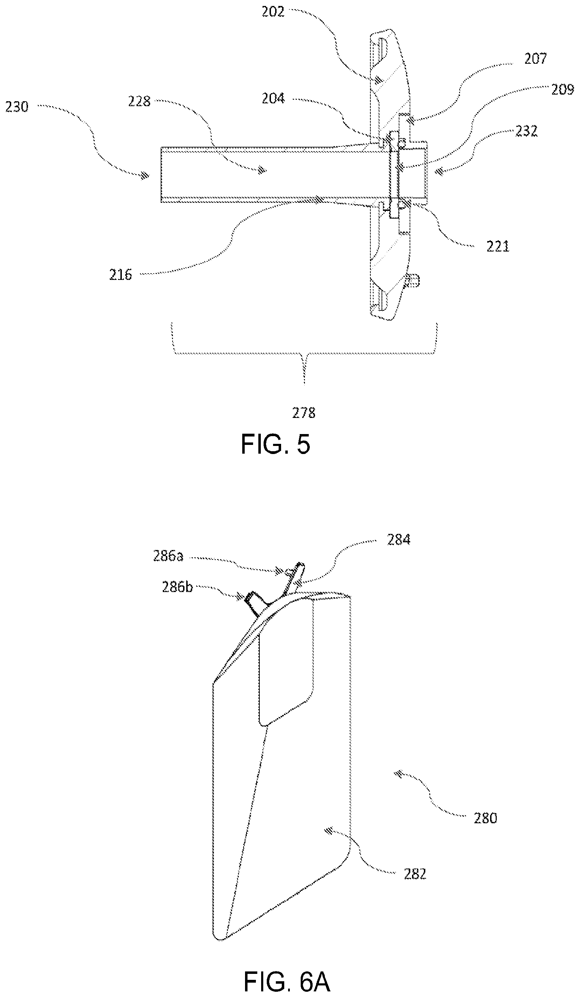

The valve cover 207 and nozzle cap 208 have been removed from FIGS. 4A and 4B to facilitate the description of the operation of opening and closing the valve appliance 200. In FIG. 4A, the gate valve 204 is in the closed position and is substantially aligned with the base 202. The gate valve is shown with cantilevered arms 263a and 263b that are formed by arm slots 265a and 265b. The ends of the cantilever arms 263a and 263b have detents 261a and 261b. The detents 261a and 261b are constrained in closed pockets 267a and 267b formed in the base 202. The detents 261a and 261b, and closed pockets 267a and 267b are configured to constrain the gate valve 204 relative to the base 202 and prevent the gate valve 204 from sliding unintentionally thereby keeping the valve appliance 200 closed. When the patient wants to open the valve appliance 200, they pull the valve handle 205 in the direction shown by arrow "O" in FIG. 4B. When the pulling force of the detents 261a and 261b against the closed pockets 267a and 267b is sufficient, the cantilever arms 263a and 263b will flex into the arm slots 255a and 265b and the detents 261a and 261b will release from the closed pockets 267a and 267b. The gate valve 204 will then slide until the detents 261a and 261b engage with the open pockets 269a and 269b that are formed in the base 202. The cantilever arms 263a and 263b will then spring back to hold the detents 261a and 261b in the open pockets 269a and 269b. In this open configuration the gate valve 204 gate opening 209 is aligned with the entry port 230, drain passage 228, base opening 231 and valve cover opening 232 such that there is a complete valve passage 278 as seen in FIG. 5. The valve appliance 200 is open in this configuration and effluent can exit the patient's body. When the patient is finished removing effluent, they apply a force to the valve handle 205 in the direction opposite arrow "O" and slide the gate valve 204 back to the closed position. In this manner the patient controls the timing of effluent exiting their body.

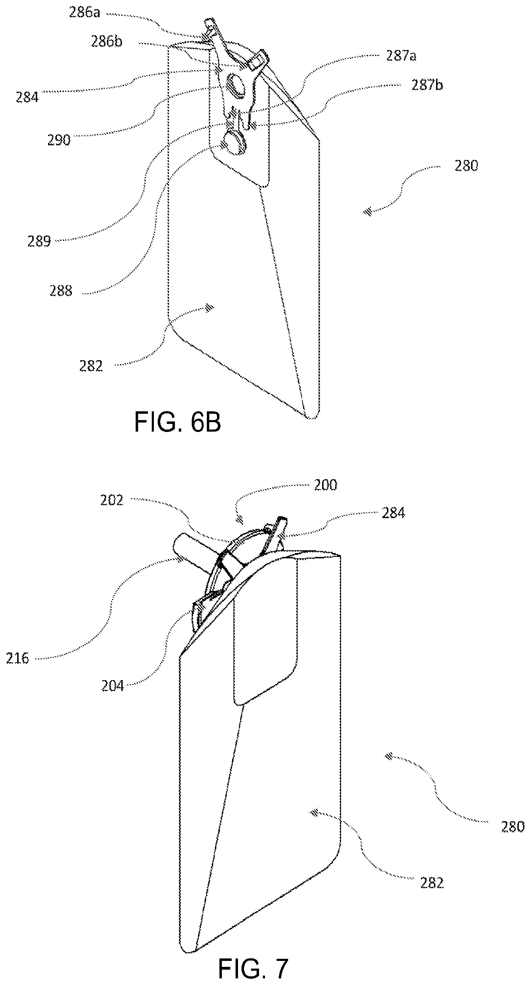

When the effluent exits the valve cover opening 232, it is preferred that the effluent be collected in a waste bag 280 such as shown in FIGS. 6A and 6B, for example. The waste bag 280 is comprised of a pouch 282 to hold the waste and a clip 284 to connect the waste bag 280 to a valve appliance such as valve appliance 200. The clip 284 is comprised of one or more hooks 286a and 286b and one or more tabs 287a and 287b that are configured to hold the waste bag 280 to the valve appliance 200. The clip 284 also contains an opening 290 that is configured to engage with the exit nozzle 226 to ensure that effluent coming out of the exit nozzle 226 goes into the pouch 282 and does not leak or spill. The clip can also include a bag cap (plug) 288 that is connected to the clip by a flexible tether 289. The bag cap 288 can be used to seal the opening 290 after the waste bag 280 has collected effluent from the valve appliance 200 and the waste bag 280 is separated from the valve appliance 200. The opening 290 can be configured with a seal to seal against the exit nozzle 226 or against the bag cap 288 to ensure effluent doesn't leak at this connection.

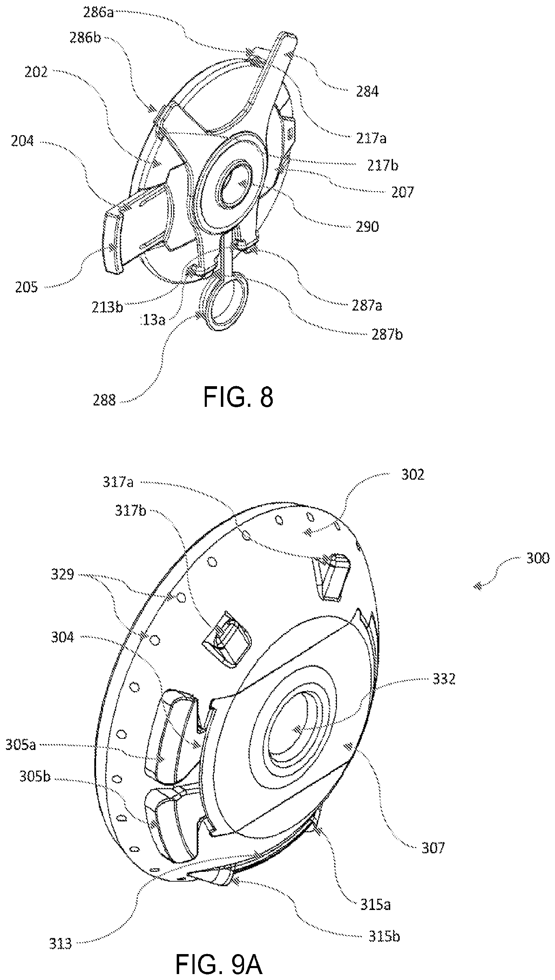

FIG. 7 shows the waste bag 280 attached to the valve appliance 200 with the valve appliance 200 in an open configuration to empty effluent from the patient through the valve appliance 200 and into the waste bag 280. In FIG. 8 the pouch 282 has been removed from the clip 284 to show details of how the waste bag 280 is connected to the valve appliance 200. The one or more tabs 287a and 287b of the clip 284 are inserted into the one or more bag clip engagement slots 213a and 213b on the base 202 and the one or more hooks 286a and 286b of the clip 284 are snapped over the one or more bag clip engagement tabs 217a and 217b. This secures the clip 284 and therefore the waste bag 280 to the valve appliance 200 and engages the waste bag 280 opening 290 with the exit nozzle 226. In use, the patient will first attach the waste bag 280 to the valve appliance 200 before opening the gate valve 204 by pulling on the valve handle 205. Then the effluent will flow through the open valve appliance 200 and into the waste bag 280. After all effluent has been removed through the patient's stoma, the patient will then push the valve handle 205 to close the gate valve 204. After the valve appliance 200 is closed, the patient will disengage the clip 284 and remove the waste bag 280 from the valve appliance 200. Then the bag cap 288 can be placed on the opening 290 of the waste bag to prevent effluent from leaking. The nozzle cap 208 can also be placed over the exit nozzle 226 of the valve appliance 200.

The base 202, gate valve 204, valve cover 207, and bag clip 284 can all be fabricated from a rigid plastic resin such as, but not limited to, polypropylene, ABS, polycarbonate, and any blend of such materials. These parts also could be manufactured from metals like aluminum or stainless steel. The substrate portion of adhesive wafer 206 can be fabricated from a flexible material such as, but not limited to, silicone, polyurethane foam or film, TPE (thermoplastic elastomer), polyethylene foam or film, PVC foam, nitrile rubber, and any blend of such materials. The adhesive portion of adhesive wafer 206 can be fabricated from either acrylic or synthetic rubber hydrocolloid adhesive or other adhesives that are skin compatible. The stem 216 can be fabricated from a flexible material such as, but not limited to, silicone, polyurethane, TPU, TPE, polyethylene. The stem 216 can be fabricated from rigid or semi-rigid plastic resins such as but not limited to polypropylene, abs, polycarbonate, and any blend of such materials. The gas-permeable filter 238 can be fabricated from materials such as but not limited to such as polyester, rayon, acrylic, polyethylene, polypropylene, cotton, and blends of such. These materials can natural hydrophobic properties or they can be treated with coatings such as but not limited to bifunctional polysiloxanes to achieve hydrophobic performance. The annular seal 216 can be fabricated from a flexible or semi-rigid material such as put not limited to silicone, polyurethane, TPU, TPE, and other elastic materials. The waste bag 280 can be fabricated from a flexible material such as but not limited to polyethylene, LDPE, HDPE, and other resin films.

In a further alternative embodiment, instead of pouch 282, clip 284 as described above may be provided on a flexible discharge tube to allow the patient to direct waste directly into a toilet. In another alternative, a flexible discharge tube may be provided with an outside diameter at one end that is sized for a slight interference fit with the inside diameter of exit nozzle 226, whereby the discharge tube may be securely inserted into the exit nozzle 226 when needed. Alternately, the inside diameter at one end of the discharge tube is sized for a slight interference fit with the outside diameter of the exit nozzle 226 to secure the discharge tube to the exit nozzle 226 to direct waste into a toilet. A flexible discharge tube of this type may be more conveniently carried by a patient in situations where the patient has access to appropriate waste disposal facilities.

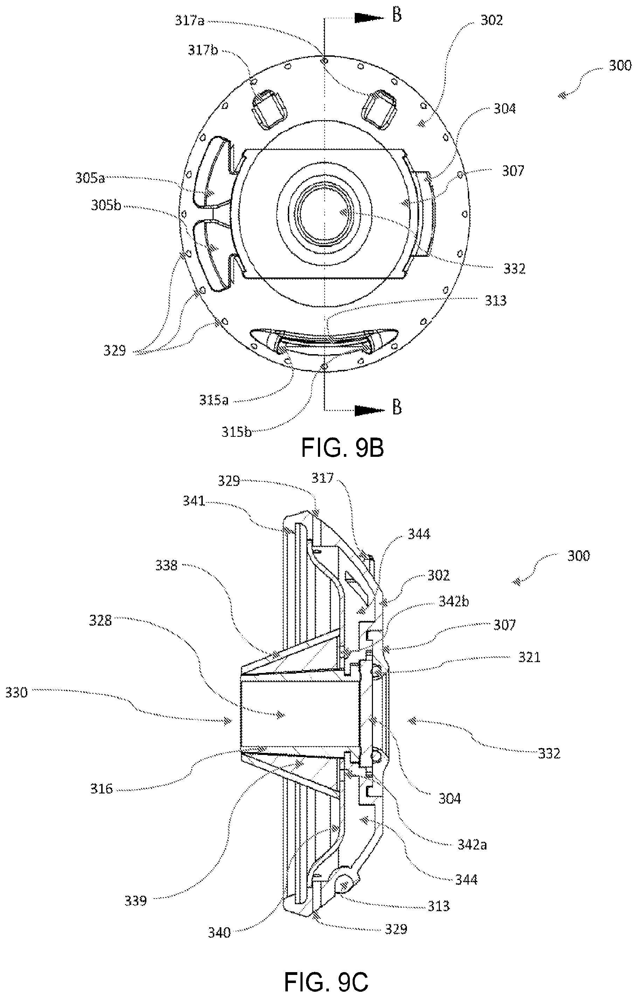

The description of the valve appliance 200 above is not intended to encompass all possible variations of valve appliances 200 covered by this disclosure. Turning now to FIGS. 9A, 9B and 9C, an alternative embodiment of a valve appliance 300 is shown. This valve appliance 300 is comprised of a base 302, a valve gate 304, a valve cover 307, a seal 321, a stem 316, and a filtration plate 340. The base 302 includes one or more bag clip engagement tabs 317a and 317b, one or more bag clip pockets 313, and one or more bag clip guides 315a and 315b to engage a waste bag clip 384 as will be described further herein. The base 302 is also includes a plurality of vents 329 to vent gases through the valve appliance 300 as will be described further herein. The valve cover 307 includes an exit port 332 to allow effluent to leave the valve appliance 300. The seal 321 is located between the gate valve 304 and the valve cover 307 to ensure that effluent does not leak between the gate valve 304 and the valve cover 307 (see FIG. 9C). The stem 316 has an entry port 330 to allow effluent to enter the drain passage 328 that passes through the center of the stem 316. The stem 316 can also seal against the gate valve 304 to ensure that effluent does not leak between the stem 316 and the gate valve 304.

The stem 316 is surrounded by a primary gas permeable filter 338 that is configured to be compressed against the inside diameter of the stoma and prevent effluent from leaking between the outer surface of the primary gas permeable filter 338 and the stoma. The stem 316 can also be surrounded by a secondary gas permeable filter 339 that is sandwiched between the stem 316 and the primary gas permeable filter 338. The secondary gas permeable filter 339 can be configured with finer filtration than the primary gas permeable filter 338. Providing more than one permeable filter can enable the valve appliance 300 to block solid material and more viscous liquid material with the primary gas permeable filter 338 and block all remaining liquid with the secondary gas permeable filter 339 while allowing gas to pass through. The valve appliance 300 is not limited to just two layers of filters. Any number of filter layers of varying filtration capabilities are possible. Having multiple layers of filtration capabilities allows liquid and solid material to be blocked and gas to pass through without the liquid and solid material clogging the first layer of filter.

It is important for the valve appliance 300 to allow the passage of gas so that the gas does not build up in the patient's digestive tract and cause uncomfortable pressure and sensations. The ability for the valve appliance 300 to release gas continuously while holding liquids and solids until the patient chooses to remove that effluent helps reduce the frequency the patient operates the valve appliance to remove effluent. Gas that passes through the primary gas permeable filter 338 and the secondary gas permeable filter 339 is directed through one or more entry ports 342a-b in the filtration plate 340. The filtration plate 340 is secured to the back side of the base 302 to form a filtration cavity 344 between the base 302 and the filtration plate 340. The filtration plate 340 can be secured to the base 302 with adhesive bonding or any other type of bonding such as ultrasonic welding. The filtration cavity 344 can be filled with an odor absorbing material including but not limited to activated charcoal. The activated charcoal will absorb odors from gases passing through the filtration cavity before the gasses exit the valve appliance 300 through the plurality of exit ports 329. In this manner the valve appliance 300 can continuously release filter gases that do not have an unpleasant odor while allowing the patient to choose when to open and close the valve appliance 300 to remove liquid and solid effluent.

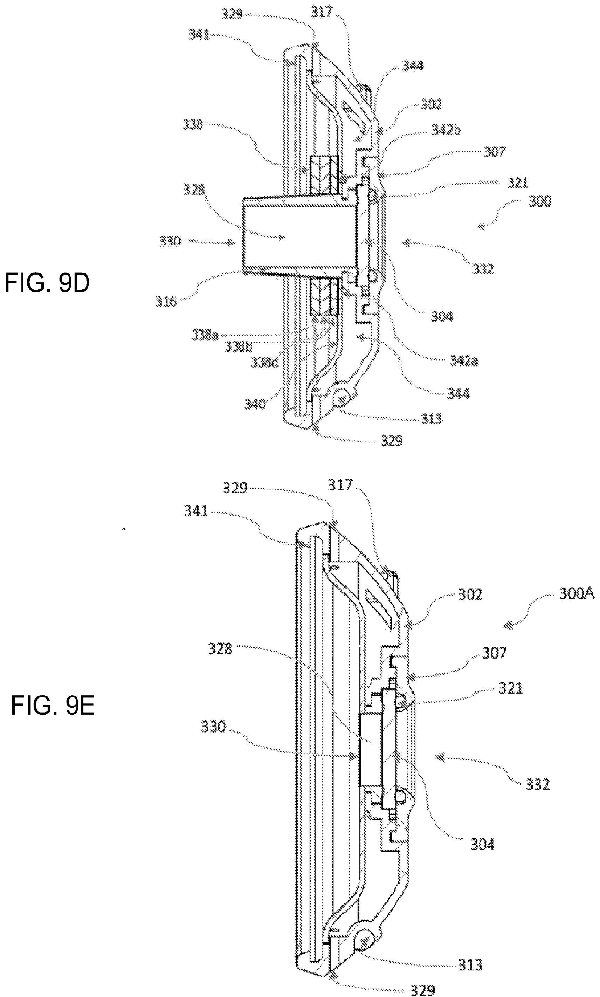

In addition to providing a seal between the stem 316 and the patient's stoma and a filter for gases, the gas permeable filter 338 also provides a soft interface between the stoma and the valve appliance 300. A soft interface is important to ensure that the valve appliance 300 that can be worn in place for several days before removal and replacement does not irritate the stoma. In addition to covering the stem 316, the secondary gas permeable filter 339, and the one or more entry ports 342a-b, the gas permeable filter 338 can also cover the outer surface of the filtration plate 340. In this manner the gas permeable filter 338 can provide a soft interface between all the surfaces of the valve appliance 300 that are in contact with the patient's stoma. An alternate soft material such as gauze dressing typically used on a wound could also be used to cover the outer surface of the filtration plate 340 instead of the gas permeable filter 338 to provide a soft interface between the valve appliance 300 and the patient's stoma. In a further alternative embodiment, illustrated in FIG. 9D, primary gas permeable filter/seal 338 is configured as an annular disk filter/seal fitted around stem 316. When configured as an annular disk filter/seal, primary filter/seal 338 may be a multi-layer structure wherein each layer is designed to filter a specific particle size. For example, a first inner layer 338a may have a filter pore size in the range of about 0.2-1.0 millimeters (example materials include- polyurethane foam; metal mesh; vinyl mesh) in order to remove macroscopic solids, a second, intermediate layer 338b may have a filter pore size in the range of about 10-50 microns (example materials include polypropylene or rayon) to capture microscopic solids, and third, outer layer 338c may have a filter pore size in the range of about 0.2 - 0.5 microns (example materials include PTFE) to prevent liquids from entering entry ports 342a,b. In this manner filter plugging may be reduced, allowing gasses to escape while continuing to seal against liquid escape into filtration cavity 344.

Valve appliance 300 may be attached to the patient using an annular disk attachment member such as adhesive wafer 206 as described above. For this purpose base 302 may be provided with annular base clip 341 configured to engage wafer clip 243, as shown, for example, in FIG. 2C (where wafer clip 243 alternatively engages base clip 241 of base 202). Alternative means of patient attachment as described above also may be used. Base 302 is also preferably formed with an interior domed shape as shown, for example, in FIG. 9C, to accommodate the patient's stoma, which may protrude from the surrounding skin surface.

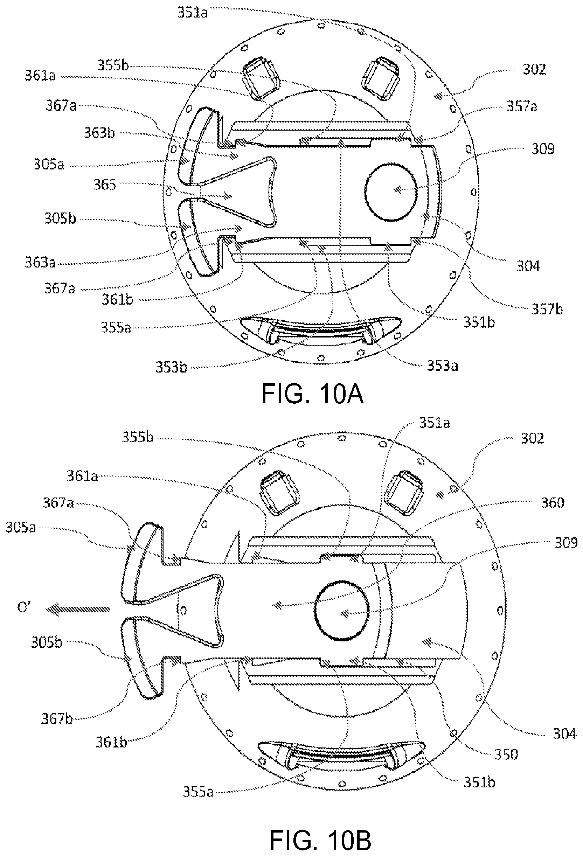

The valve cover 307 and seal 321 have been removed from FIGS. 10A and 10B to illustrate the operation of the valve appliance 300. The valve gate 304 is comprised of a body 360, gate guides 351a and 351b on either side of the body 360, cantilever arms 363a and 363b at one end of the body 360, and valve handles 305a and 305b on the ends of the cantilever arms 363a and 363b. The valve gate 304 is held constrained in a closed position relative to the base 302 by lateral stops 361a and 361b that project from the cantilever arms 363a and 363b that butt against stop ledges 367a and 367b on the base 302. To open the gate valve 304, the patient squeezes the valve handles 305a and 305b towards each other and pulls the gate valve 304 in the direction of the "Arrow O‴. Squeezing the valve handles 305a and 305b towards each other deflects cantilever arms 363a and 363b which in turn displaces the lateral stops 361a and 361b such that they are free of the stop ledges 367a and 367b. The gate guides 351a and 351b slide against the lateral sides 353a and 353b of the base slot 350 to keep the gate valve moving in the direction of the Arrow O' until the gate guides 351a and 351b butt against the slot ledges 355a and 355b. When the gate guides 351a and 351b butt against the slot ledges 355a and 355b the gate opening 309 through the gate valve 304 is aligned with the drain passage 328 and effluent can exit the patient. The patient can push the valve handles 305a and 305b in the direction opposite Arrow O' to close the gate valve 304. Although the valve appliance 300 illustrated in FIGS. 9A through 10B only has one set of lateral stops 361a and 361b and stop ledges 367a and 367b that hold the gate valve 304 locked until the patient wants to open the valve appliance 300 to remove effluent, the valve appliance 300 could also be comprised of another set of stops and ledges to hold the gate valve 304 open until the patient wanted to close the valve appliance 300. For example, the closed pockets 267a-b and the open pockets 269a-b engaging the detents 261a-b in valve appliance 200 illustrate locking the gate valve 200 in both closed and open positions.

In a further alternative, as illustrated in FIG. 9E, valve appliances according to the present disclosure may be configured without a stem or gas vent. As one such example, alternative valve appliance 300A is structured substantially the same as valve appliance 300, but without stem 316 and without the ports, filters and passages associated with the gas vent.

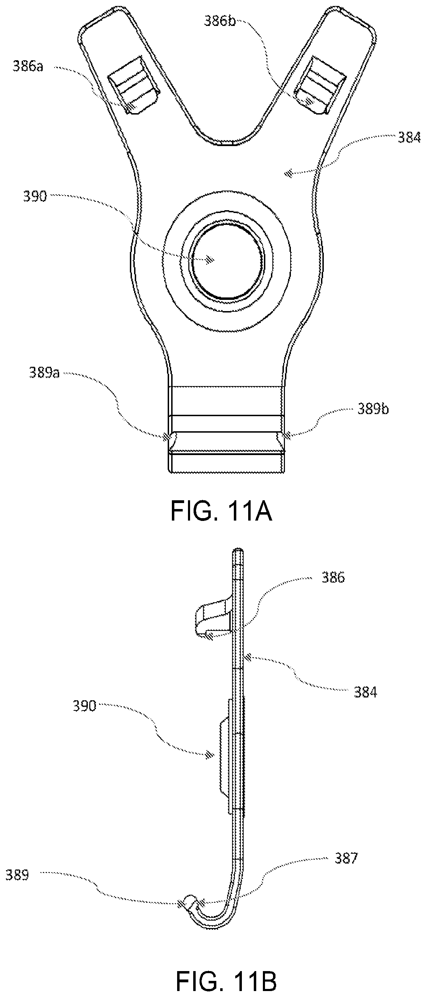

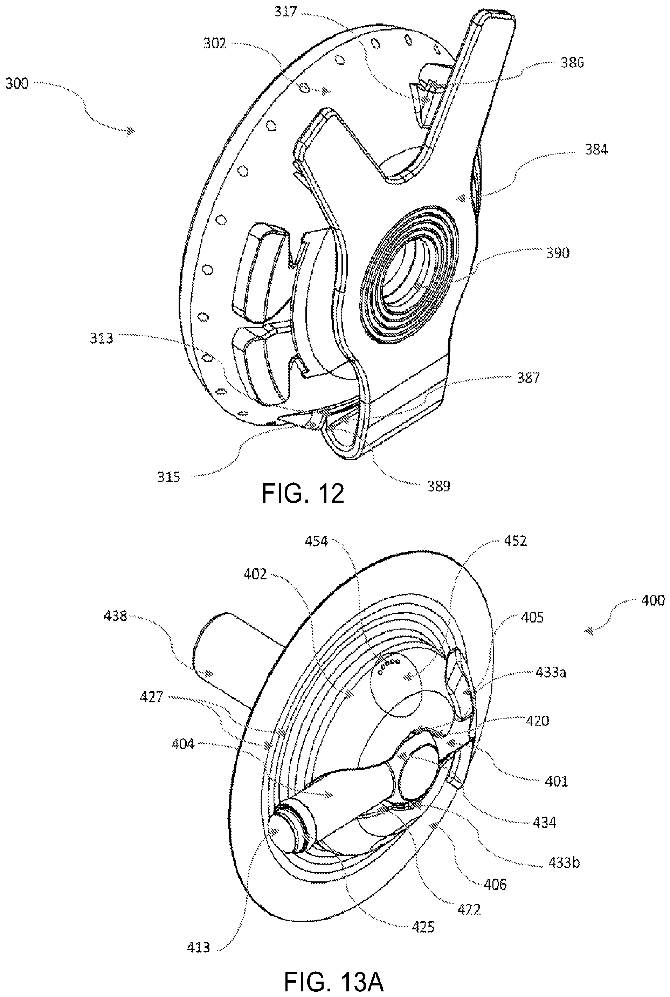

FIGS. 11A and 11B illustrate an alternative embodiment of a waste bag clip 384 with the pouch 282 that would be connected to the waste bag clip 384 not shown so as to highlight the features of the waste bag clip 384. The waste bag clip 384 is comprised of one or more hooks 386a and 386b and one or more tabs 387 that are configured to hold the waste bag clip 384 to the valve appliance 300. The waste bag clip 384 also has an opening 390 that is configured to engage with the exit port 332 of the valve appliance 300 to ensure that effluent coming out of the exit port 332 goes into the pouch 282 and does not leak or spill. The one or more tabs 387 have one or more guiding surfaces 389a and 389b configured to align the one or more tabs 387 with the clip pocket 313 on the valve appliance 300 base 302 (See FIGS. 9A-C and 12). To attach the waste bag clip 384 to the base 302, the one or more tabs 387 of the waste bag clip 384 is placed into the clip pocket 313 on the base 302. The guiding surfaces 389a and 389b on the one or more tabs 387 interface with the sides 315a and 315b of the clip pocket 313 to facilitate the placement of the one or more tabs 387 into the clip pocket 313. The waste bag clip 384 is then pushed against the base 302 until the one or more hooks 386a and 386b of the waste bag clip 384 catch the one or more bag clip engagement tabs 317a and 317b on the base 302. Once the waste bag clip 384 and with it the waste bag 28 is securely attached to the valve appliance 300, the patient can open the valve gate 304 to remove effluent from their body. The valve appliance 300 or 200 can be used without a waste bag clip to remove effluent from the body into a toilet of similar receptacle but use of the waste bag 280 can make the process easier and cleaner for the patient.

The base 302, gate valve 304, valve cover 307, and bag clip 384, and filtration plate 340 can all be fabricated from similar materials as were described herein for the base 202, gate valve 204, valve cover 207, and bag clip 284 Similarly, the stem 316 can be fabricated from similar materials as were described herein for the stem 216. It is also to be noted that while stem 316 is shown in this example to be shorter than the stems of other embodiments, for example stem 216, stems of varying lengths or no stem may be utilized with any of the disclosed embodiments. Where a stem is included, it may vary in length from having an inner end lying approximately co-planar with the inside surface of the base to the longer stems shown in various embodiments. One factor bearing on stem length would be patient comfort. Specific vent designs also may require longer or shorter stems.

FIGS 13A-E illustrate another alternate embodiment of a valve appliance 400. The valve appliance 400 is comprised of a base 402, an adhesive wafer 406, a ball valve 434, a valve lock 401, and a stem 416. The base 402 is configured on the front side with a ball cavity 420 and a tube cavity 422. The ball cavity 420 is configured with two side slots 433a-b. The ball cavity is configured to accept the ball valve 434 and the side slots 433a-b are configured to accept the ball axels 435a-b. The ball valve 434 pivots relative to the base 402 around the axis formed by the ball axels 435a-b. The base is also comprised of one or more filter cavities 453a-b. The one or more filter cavities 453a-b are covered with one or more filter cavity covers 452a-b. The one or more filter cavity covers 452a-b contain order absorbing material inside the one or more filter cavities 453a-b. The one or more filter cavity covers 452a-b can be attached to the base 402 with adhesive bonding or any other type of bonding including ultrasonic welding, solvent bonding, insert molding, and the like. The one or more filter cavity covers 452a-b contain a plurality of openings 454 to allow gases to pass through the odor absorbing material in the one or more filter cavities 453a-b and out of the valve appliance 400. The base 402 is configured on the back side with one or more ribs 494 that interface with one or more grooves 492 on the adhesive wafer to securely attach the base 402 to the adhesive wafer 406. The ribs 494 and grooves 492 can be attached with adhesive bonding or any other type of bonding including ultrasonic welding, solvent bonding, insert molding, and the like.

The adhesive wafer 406 is comprised of one or more grooves 427. The one or more grooves 427 can be on either the front surface or the back surface of the adhesive wafer 406 and provide for a more flexible adhesive wafer 406 to optimize the adhesive wafer's 406 conformation and adherence to the patient's skin. The back side of the adhesive wafer 405 is comprised of a skin compatible adhesive to ensure the valve appliance 400 can be adhered to the patient's skin for several days to keep the valve appliance 400 in position without irritating the patient's skin.

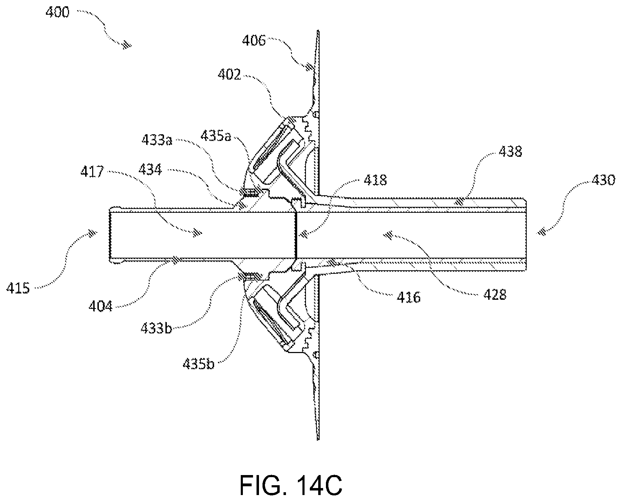

The ball valve 434 is also comprised of an exit nozzle 404 at the end of which is the exit port 415. The ball valve 434 also has a bottom port 418 which is in communication with the exit port 516 by means of a nozzle passage 417 (See FIG. 14C).

The valve lock 401 is comprised of an elongated rod 403 with an end face 413 at one end and a lock handle 405 at the other end. The valve lock 401 also has a wiping edge 425 adjacent to the end face 413. The elongated rod 403 fits in the nozzle passage 417 of the ball valve 434 with the wiping edge 425 extending past the exit nozzle 404. The outer diameter of the wiping edge 425 is greater than the inside diameter of the nozzle passage 417 and thereby holds the valve lock 401 inside the ball valve 434 with the valve lock 401 preventing the ball valve 434 from rotating thereby locking the ball valve 434 and keeping the valve appliance 400 closed. The valve lock 401 also has a guide channel 410 with a guide stop 414 near the end face 413. The guide channel is configured to slide over a stop boss 412 on the base 402. When the valve lock 401 is actuated as will be described further herein, the stop boss 412 prevents the valve lock 401 from rotating, and the guide stop 414 prevents the valve lock 401 from being separated from the valve appliance 400.

The stem 416 is attached to the base 402 and is covered by a gas permeable filter 438. The back end of the stem 416 has an entry port 430 that allows effluent to enter the drain passage 428. The front end of the stem 416 is compressed against the ball valve 434 to prevent effluent from leaking from the stem when the valve appliance 400 is in the locked position. The gas permeable filter 438 is comprised of one or more wicks 439 that pass-through slots 440 in the base 402 to the one or more filter cavities 453a-b.

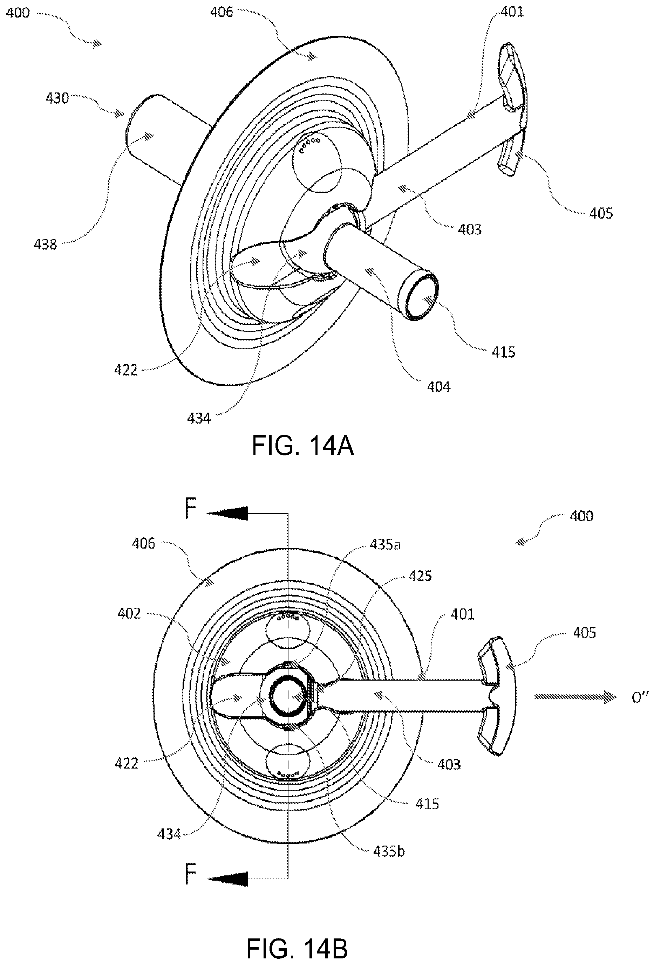

FIGS. 14A, 14B and 14C show the valve appliance 400 in an open position. To open the valve appliance 400, the patient first holds the lock handle 405 and slides the valve lock 401 in the direction of Arrow O". This action pulls the wiper edge 425 into the exit port 415 and through the nozzle passage 417 of the ball valve 434. When the valve lock 401 is completely withdrawn from the ball valve 434, the guide stop 414 contacts the stop boss 412 of the base 402 preventing additional translation of the valve lock 401. The patient then holds the exit nozzle 404 of the ball valve 434 and pivots the ball valve 434 about the axis formed by the ball axels 435a-b. This moves the ball valve 434 from the horizontal locked position to a vertical open position. In this open position, there is a continuous opening created starting from the entry port 430 on the end of the stem 416, through the drain passage 428 in the stem 416, to the bottom port 418 on the ball valve 434, through the nozzle passage 417 in the exit nozzle 404, and ending at the exit port 415. In this manner effluent can enter the valve appliance 400 through the entry port 430 and leave through the exit port 415 and the patient can remove effluent from their body.

After removing all effluent, the patient can rotate the ball valve 434 back to a horizontal position closing the valve appliance 400. Then the patient can slide the valve lock 401 back into the ball valve 434. The wiper edge 425 is pushed into the bottom port 418, through the nozzle passage 417 in the exit nozzle 404, and out the exit port 415. This actuation of the valve lock 401 with the wiper edge 425 near the end face 413 cleans out any effluent that maybe still present in the nozzle passage 417 so that the ball valve 434 is clean until the next time the patient wants to actuate and open the valve appliance 400.

The base 402, ball valve 404, cavity covers 452a-b, and valve lock 401, can all be fabricated from similar materials as were described herein for the base 202, gate valve 204, and valve cover 207. Similarly, the wafer 406, the stem 416, gas permeable filter 438, and wiping edge 425 can be fabricated from similar materials as were described herein for the wafer 206 and the stem 216, gas permeable filter 238, and annular seal 221, respectively.

In further alternative embodiments, valve appliances may be configured substantially as described hereinabove, but without a gas vent. The need for gas venting in some cases may be correlated to patient personal habits such as frequency of evacuation and diet. Patients that do not require a gas vent may therefore prefer the greater control that could be afforded by a ventless valve appliance. Further, in some embodiments it may be preferable to not include the stem component while otherwise configuring valve appliance embodiments in accordance with the embodiments described herein. Removal of the stem component for some patients may increase comfort of the device. Additionally, as one purpose of the stem in some embodiments is to create functionally separate pathways for evacuation of gas versus liquid/solid wastes, it may be desirable to omit the stem component in unvented embodiments.

In view of the foregoing, the improvements discussed herein can facilitate discharge of waste from stoma. The embodiments employ materials that self-seal with surfaces inside of the patient's body. This feature eliminates the need for the patient to interact with the device to properly affix it in position so as to avoid leaks or other potential mistakes that can allow waste to inadvertently discharge from the stoma.

The foregoing has been a detailed description of illustrative embodiments of the disclosure. It is noted that in the present specification and claims appended hereto, conjunctive language such as is used in the phrases "at least one of X, Y and Z" and "one or more of X, Y, and Z," unless specifically stated or indicated otherwise, shall be taken to mean that each item in the conjunctive list can be present in any number exclusive of every other item in the list or in any number in combination with any or all other item(s) in the conjunctive list, each of which may also be present in any number. Applying this general rule, the conjunctive phrases in the foregoing examples in which the conjunctive list consists of X, Y, and Z shall each encompass: one or more of X; one or more of Y; one or more of Z; one or more of X and one or more of Y; one or more of Y and one or more of Z; one or more of X and one or more of Z; and one or more of X, one or more of Y and one or more of Z.

Features of each of the various embodiments described above may be combined with features of other described embodiments as appropriate in order to provide a multiplicity of feature combinations in associated new embodiments. Furthermore, while the foregoing describes a number of separate embodiments, what has been described herein is merely illustrative of the application of the principles of the present disclosure. Additionally, although particular methods herein may be illustrated and/or described as being performed in a specific order, the ordering is highly variable within ordinary skill to achieve aspects of the present disclosure. Accordingly, this description is meant to be taken only by way of example, and not to otherwise limit the scope of this disclosure.

Exemplary embodiments have been disclosed above and illustrated in the accompanying drawings. It will be understood by those skilled in the art that various changes, omissions and additions may be made to that which is specifically disclosed herein without departing from the scope of the present disclosure.

## Claims

1. A stoma valve appliance (100, 200, 300, 400), comprising:
an annular support member configured to surround a patient stoma and having an inner, skin facing side and an outer side;
a base member (302), removably attachable over a center of the annular support member, said base member defining a first solids and liquids flow path and a second gas vent flow path through the base member, each said flow path having at least one opening on an inner side of the base member; **characterized by**
a valve disposed in the base member configured to control flow through said first flow path, the valve having an open position permitting flow and a closed position preventing flow and being manipulable between the open and closed position by the patient;
a stem (316) with an inlet and an outlet projecting inwardly from the base member configured to be received in the patient stoma, the stem having sufficient length to extend through the center of the annular support member to position the inlet in the stoma when the base member is mounted on the annular support member with the outlet at an opposite end communicating with the first flow path opening in the base member; and
a gas permeable filter seal (338) around the stem covering the at least one second gas vent flow path opening, whereby gasses from the stoma may be vented through said second flow path, while liquids and solids are prevented from entering said second flow path.

2. The stoma valve appliance of claim 1, wherein the base member (302) has an inwardly facing concave surface shaped to accommodate a patient stoma protruding through the center of the annular support member.

3. The stoma valve appliance of claim 1 or claim 2, further comprising means for affixing the annular support member to the patient's skin disposed on said annular support member inner, skin facing side, and
wherein the means for affixing preferably comprises an adhesive material.

4. The stoma valve appliance of any of claims 1-3, wherein the gas permeable filter seal (338) comprises plural filter layers with different pore sizes, and
wherein the gas permeable filter seal preferably comprises a sleave at least partially surrounding the stem configured to extend into the stoma between the stem and the patient; or
wherein the gas permeable filter seal comprises an annular disk filter member through which the stem extends.

5. The stoma valve appliance of any of claims 1-4, wherein the base member gas vent flow path terminates in at least one exhaust port opposite the inner side of the base member a (302) and the base member defines at least one filtration cavity in the gas vent flow path between the at least one inner side opening and at least one exhaust port, and
wherein the at least one filtration cavity preferably contains an odor absorbing material.

6. The stoma valve appliance of any of claims 1-5, wherein the annular support member and base member are attached by being pressed together.

7. The stoma valve appliance of claim 6, wherein the annular support member and base member each have facing engagement surfaces that engage and fix said members together when pressed together.

8. The stoma valve appliance of claim 7, wherein said facing engagement surfaces are resilient hook shapes; or
wherein said facing engagement surfaces comprise interlocking ribs and grooves.

9. The stoma valve appliance of any of claims 1-5, wherein the annular support member and base member (302) are attachable with a threaded connection.

10. The stoma valve appliance of any of claims 1-9, further comprising an exit nozzle terminating the first flow path through the base member opposite the inner side opening.

11. The stoma valve appliance of claim 10, further comprising:
a waste collection bag, wherein the waste collection bag comprises -
a pouch;
a connector sealed to the pouch having an opening configured to mate with the exit nozzle to receive solids and liquids therefrom; and
first engagement means disposed on the connector; and
second engagement means disposed on the base member configured to engage said first engagement means to secure the waste collection bag to the base member with the exit nozzle mated to the connector opening.

12. The stoma valve appliance of any of claims 1-11, wherein the valve is a gate valve; and
wherein the gate valve preferably comprises a valve gate slidably mounted in the base member, and
wherein the valve gate preferably includes a detent locking mechanism engageable with the base member to lock the valve gate in the closed position, and
wherein the detent locking mechanism preferably forms a user manipulable handle on the valve gate.

13. The stoma valve appliance of claims 1-12, wherein the valve is a ball valve.

14. The stoma valve appliance of claim 13, wherein the ball valve comprises a ball member and a nozzle member with the ball member rotatably received in a spherical recess in the base member to rotate from the closed position with the nozzle lying against the base member and the open position with the nozzle extending outwardly from the base member.

15. The stoma valve appliance of claim 14, wherein the ball valve further comprises a locking plunger configured to slide along the base member and into the ball valve nozzle when in the closed position preventing rotation of the ball valve to the open position and forcing wastes from the nozzle.

## Patentansprüche

1. Eine Stoma-Ventilvorrichtung (100, 200, 300, 400), die Folgendes umfasst:
ein ringförmiges Stützelement, das so ausgebildet ist, dass es ein Stoma eines Patienten umgibt, und das eine der Haut zugewandte Innenseite sowie eine Außenseite aufweist;
ein Basiselement (302), das abnehmbar über einer Mitte des ringförmigen Stützelements anbringbar ist, wobei das Basiselement einen ersten Strömungsweg für Feststoffe und Flüssigkeiten und einen zweiten Entlüftungsströmungsweg für Gas durch das Basiselement definiert, wobei jeder Strömungsweg mindestens eine Öffnung an einer Innenseite des Basiselements aufweist; **dadurch gekennzeichnet, dass**
ein im Basiselement angeordnetes Ventil dazu ausgelegt ist, den Durchfluss durch den ersten Strömungsweg zu steuern, wobei das Ventil eine den Durchfluss erlaubende offene Stellung und eine den Durchfluss verhindernde geschlossene Stellung aufweist und vom Patienten zwischen der offenen Stellung und der geschlossenen Stellung betätigt werden kann;
ein Schaft (316) mit einem Einlass und einem Auslass nach innen aus dem Basiselement herausragt und so ausgebildet ist, dass er im Stoma des Patienten aufgenommen werden kann, wobei der Schaft eine ausreichende Länge aufweist, um sich durch die Mitte des ringförmigen Stützelements zu erstrecken, um den Einlass im Stoma zu positionieren, wenn das Basiselement auf dem ringförmigen Stützelement montiert ist, wobei der Auslass an einem gegenüberliegenden Ende mit der ersten Strömungswegöffnung im Basiselement in Verbindung steht; und
eine gasdurchlässige Filterdichtung (338) um den Schaft herum mindestens eine zweite Gasentlüftungs-Strömungswegöffnung abdeckt, wodurch Gase aus dem Stoma durch den genannten zweiten Strömungsweg entlüftet werden können, während Flüssigkeiten und Feststoffe daran gehindert werden, in den genannten zweiten Strömungsweg einzudringen.

2. Die Stoma-Ventilvorrichtung nach Anspruch 1, wobei das Basiselement (302) eine nach innen gerichtete, konkave Oberfläche aufweist, die so geformt ist, dass sie ein Patientenstoma aufnimmt, das durch die Mitte des ringförmigen Stützelements ragt.

3. Die Stoma-Ventilvorrichtung nach Anspruch 1 oder 2, die ferner Mittel zum Befestigen des ringförmigen Stützelements an der Haut des Patienten umfasst, die auf der inneren, der Haut zugewandten Seite des ringförmigen Stützelements angeordnet sind, und
wobei die Mittel zum Befestigen vorzugsweise ein Klebematerial umfassen.

4. Die Stoma-Ventilvorrichtung nach einem der Ansprüche 1 bis 3, wobei die gasdurchlässige Filterdichtung (338) mehrere Filterschichten mit unterschiedlichen Porengrößen umfasst und
wobei die gasdurchlässige Filterdichtung vorzugsweise eine Hülse umfasst, die den Schaft zumindest teilweise umgibt und so ausgebildet ist, dass sie sich zwischen dem Schaft und dem Patienten **in** das Stoma erstreckt; oder
wobei die gasdurchlässige Filterdichtung ein ringförmiges Scheibenfilterelement umfasst, durch das sich der Schaft erstreckt.

5. Die Stoma-Ventilvorrichtung nach einem der Ansprüche 1 bis 4, wobei der Gasentlüftungsströmungsweg des Basiselements **in** mindestens einer Auslassöffnung endet, die der Innenseite des Basiselements (302) gegenüberliegt, und das Basiselement mindestens einen Filterhohlraum im Gasentlüftungsströmungsweg zwischen der mindestens einen Innenseitenöffnung und der mindestens einen Auslassöffnung definiert, und
wobei der mindestens eine Filterhohlraum vorzugsweise ein geruchsabsorbierendes Material enthält.

6. Die Stoma-Ventilvorrichtung nach einem der Ansprüche 1 bis 5, wobei das ringförmige Stützelement und das Basiselement durch Zusammenpressen befestigt sind.

7. Die Stoma-Ventilvorrichtung nach Anspruch 6, wobei das ringförmige Stützelement und das Basiselement jeweils einander zugewandte Eingriffsflächen aufweisen, die beim Zusammenpressen ineinandergreifen und die Elemente miteinander fixieren.

8. Die Stoma-Ventilvorrichtung nach Anspruch 7, wobei die einander zugewandten Eingriffsflächen elastische Hakenformen aufweisen; oder
wobei die einander zugewandten Eingriffsflächen ineinandergreifende Rippen und Nuten umfassen.

9. Die Stoma-Ventilvorrichtung nach einem der Ansprüche 1 bis 5, wobei das ringförmige Stützelement und das Basiselement (302) mittels einer Schraubverbindung befestigbar sind.

10. Die Stoma-Ventilvorrichtung nach einem der Ansprüche 1 bis 9, die ferner eine Auslassdüse umfasst, die den ersten Strömungsweg durch das Basiselement gegenüber der inneren Seitenöffnung abschließt.

11. Die Stoma-Ventilvorrichtung nach Anspruch 10, die ferner Folgendes umfasst:
einen Abfallsammelbeutel, wobei der Abfallsammelbeutel Folgendes umfasst:
einen Beutel;
einen mit dem Beutel abgedichteten Anschluss, der eine Öffnung aufweist, die so ausgebildet ist, dass sie mit der Auslassdüse **in** Eingriff kommt, um Feststoffe und Flüssigkeiten von dieser aufzunehmen; und
eine erste Eingriffseinrichtung, die an dem Verbinder angeordnet ist; und
eine zweite Eingriffseinrichtung, die an dem Basiselement angeordnet und so ausgebildet ist, dass sie mit der ersten Eingriffseinrichtung **in** Eingriff kommt, um den Abfallsammelbeutel an dem Basiselement zu befestigen, wobei die Auslassdüse mit der Öffnung des Verbindungsstücks **in** Eingriff steht.

12. Die Stoma-Ventilvorrichtung nach einem der Ansprüche 1 bis 11, wobei das Ventil ein Absperrventil ist; und
wobei das Absperrventil vorzugsweise einen im Basiselement verschiebbar gelagerten Ventilschieber umfasst, und
wobei das Absperrventil vorzugsweise einen Rastverriegelungsmechanismus aufweist, der mit dem Basiselement **in** Eingriff gebracht werden kann, um das Absperrventil **in** der geschlossenen Position zu verriegeln, und
wobei der Rastverriegelungsmechanismus vorzugsweise einen vom Benutzer betätigbaren Griff am Ventilanschluss bildet.

13. Die Stoma-Ventilvorrichtung nach den Ansprüchen 1 bis 12, wobei das Ventil ein Kugelventil ist.

14. Die Stoma-Ventilvorrichtung nach Anspruch 13, wobei das Kugelventil ein Kugelelement und ein Düsenelement umfasst, wobei das Kugelelement drehbar **in** einer kugelförmigen Aussparung im Basiselement aufgenommen ist, um sich aus der geschlossenen Position, **in** der die Düse am Basiselement anliegt, **in** die offene Position zu drehen, **in** der die Düse vom Basiselement nach außen ragt.

15. Die Stoma-Ventilvorrichtung nach Anspruch 14, wobei das Kugelventil ferner einen Verriegelungsstößel umfasst, der so ausgebildet ist, dass er in der geschlossenen Position entlang des Basiselements in die Düse des Kugelventils gleitet, wodurch eine Drehung des Kugelventils in die geöffnete Position verhindert und Abfallstoffe aus der Düse gedrückt werden.

## Revendications

1. Dispositif de valve de stomie (100, 200, 300, 400), comprenant :
un élément de support annulaire configuré pour entourer une stomie d'un patient et présentant un côté intérieur faisant face à la peau et un côté extérieur ;
un élément de base (302), pouvant être attaché de manière amovible sur un centre de l'élément de support annulaire, ledit élément de base définissant une première voie d'écoulement pour matières solides et liquides et une seconde voie d'écoulement pour évacuation de gaz à travers l'élément de base, chaque dite voie d'écoulement présentant au moins une ouverture sur un côté intérieur de l'élément de base ; **caractérisé par**
une valve disposée dans l'élément de base configurée pour réguler un écoulement à travers ladite première voie d'écoulement, la valve présentant une position ouverte permettant l'écoulement et une position fermée empêchant l'écoulement et étant manipulable entre la position ouverte et la position fermée par le patient ;
une tige (316) présentant une entrée et une sortie faisant saillie vers l'intérieur depuis l'élément de base configurée pour être reçue dans la stomie du patient, la tige présentant une longueur suffisante pour s'étendre à travers le centre de l'élément de support annulaire de sorte à positionner l'entrée dans la stomie lorsque l'élément de base est monté sur l'élément de support annulaire avec la sortie au niveau d'une extrémité opposée communiquant avec l'ouverture de la première voie d'écoulement dans l'élément de base ; et
un joint de filtre perméable aux gaz (338) autour de la tige couvrant la au moins une ouverture de la seconde voie d'écoulement pour évacuation de gaz, grâce à quoi des gaz provenant de la stomie peuvent être évacués à travers ladite seconde voie d'écoulement, tandis que des matières liquides et solides sont empêchées de pénétrer dans ladite seconde voie d'écoulement.

2. Dispositif de valve de stomie selon la revendication 1, dans lequel l'élément de base (302) présente une surface concave orientée vers l'intérieur façonnée pour recevoir une stomie d'un patient dépassant depuis le centre de l'élément de support annulaire.

3. Dispositif de valve de stomie selon la revendication 1 ou la revendication 2, comprenant en outre un moyen permettant de fixer l'élément de support annulaire sur la peau du patient disposé sur le côté intérieur faisant face à la peau dudit élément de support annulaire, et
où le moyen de fixation comprend de préférence un matériau adhésif.

4. Dispositif de valve de stomie selon l'une quelconque des revendications 1 à 3, dans lequel le joint de filtre perméable aux gaz (338) comprend plusieurs couches de filtre de différentes porosités, et
où le joint de filtre perméable aux gaz comprend de préférence **un** manchon entourant au moins partiellement la tige configuré pour s'étendre dans la stomie entre la tige et le patient ; ou
où le joint de filtre perméable aux gaz comprend **un** élément de filtre en disque annulaire à travers lequel la tige s'étend.

5. Dispositif de valve de stomie selon l'une quelconque des revendications 1 à 4, dans lequel la voie d'écoulement pour évacuation de gaz de l'élément de base se termine en au moins **un** orifice d'évacuation opposé au côté intérieur de l'élément de base (302) et l'élément de base définit au moins une cavité de filtration dans la voie d'écoulement pour évacuation de gaz entre la au moins une ouverture du côté intérieur et le au moins **un** orifice d'évacuation , et
où la au moins une cavité de filtration contient de préférence **un** matériau absorbant les odeurs.

6. Dispositif de valve de stomie selon l'une quelconque des revendications 1 à 5, dans lequel l'élément de support annulaire et l'élément de base sont attachés en étant pressés ensemble.

7. Dispositif de valve de stomie selon la revendication 6, dans lequel l'élément de support annulaire et l'élément de base ont chacun des surfaces d'engagement se faisant face qui s'engagent et fixent lesdits éléments ensemble lorsqu'ils sont pressés ensemble.

8. Dispositif de valve de stomie selon la revendication 7, dans lequel lesdites surfaces d'engagement se faisant face sont des formes de crochets résilients ; ou
où lesdites surfaces d'engagement se faisant face comprennent des nervures et des rainures s'interverrouillant.

9. Dispositif de valve de stomie selon l'une quelconque des revendications 1 à 5, dans lequel l'élément de support annulaire et l'élément de base (302) peuvent être attachés par **un** raccord fileté.

10. Dispositif de valve de stomie selon l'une quelconque des revendications 1 à 9, comprenant en outre une buse de sortie terminant la première voie d'écoulement à travers l'élément de base à l'opposé de l'ouverture du côté intérieur.

11. Dispositif de valve de stomie selon la revendication 10, comprenant en outre :
**un** sac de collecte de déchets, où le sac de collecte de déchets comprend
une poche ;
**un** raccord scellé sur la poche présentant une ouverture configurée pour être couplée à la buse de sortie pour recevoir des matières solides et liquides provenant de cette dernière ; et
**un** premier moyen d'engagement disposé sur le raccord ; et
**un** second moyen d'engagement disposé sur l'élément de base configuré pour s'engager avec ledit premier moyen d'engagement pour fixer le sac de collecte de déchets à l'élément de base avec la buse de sortie couplée à l'ouverture du raccord.

12. Dispositif de valve de stomie selon l'une quelconque des revendications 1 à 11, dans lequel la valve est une valve à clapet ; et
où la valve à clapet comprend de préférence **un** clapet de valve monté avec possibilité de coulissement dans l'élément de base, et
où le clapet de valve inclut de préférence **un** mécanisme de verrouillage par encliquetage pouvant s'engager avec l'élément de base pour verrouiller le clapet de valve dans la position fermée, et
où le mécanisme de verrouillage par encliquetage forme de préférence une poignée manipulable par l'utilisateur sur le clapet de valve.

13. Dispositif de valve de stomie selon les revendications 1 à 12, dans lequel la valve est une valve à bille.

14. Dispositif de valve de stomie selon la revendication 13, dans lequel la valve à bille comprend **un** élément de bille et **un** élément de buse, l'élément de bille étant reçu avec possibilité de rotation dans **un** évidement sphérique dans l'élément de base pour tourner de la position fermée où la buse se trouve contre l'élément de base à la position ouverte où la buse s'étend vers l'extérieur depuis l'élément de base.

15. Dispositif de valve de stomie selon la revendication 14, dans lequel la valve à bille comprend en outre **un** plongeur de verrouillage configuré pour coulisser le long de l'élément de base et jusque dans la buse de valve à bille lorsqu'il est dans la position fermé empêchant la rotation de valve à bille vers la position ouverte et forçant les déchets à sortir par la buse.
